# EUROPÄISCHE PATENTSCHRIFT

(11) **EP 1 896 423 B1**
(45) Veröffentlichungstag und Bekanntmachung des Hinweises auf die Patenterteilung: **10.08.2011**
(21) Anmeldenummer: 06743124.7
(22) Anmeldetag: 07.06.2006
(51) Int. Cl.: C07D 231/16

(54) **VERFAHREN ZUM HERSTELLEN VON CARBOXAMIDEN**
METHOD FOR PRODUCING CARBOXAMIDES
PROCEDE DE PRODUCTION DE CARBOXAMIDES

(30) Priorität: 18.06.2005 DE 102005028294
(43) Veröffentlichungstag der Anmeldung: 12.03.2008
(73) Patentinhaber: Bayer CropScience AG, 40789 Monheim (DE)
(72) Erfinder: NEEFF, Arnd, 58256 Ennepetal (DE); PAZENOK, Sergiy, 42699 Solingen (DE)
(86) Internationale Anmeldenummer: PCT/EP2006/005436
(87) Internationale Veröffentlichungsnummer: WO 2006/136288

(56) Entgegenhaltungen:
- EP-A- 0 776 889
- ROBERT T. LALONDE, ET AL.: JOURNAL OF ORGANIC CHEMISTRY, Bd. 35, Nr. 3, 1970, Seiten 771-774, XP002395196

## Beschreibung

Die vorliegende Erfindung betrifft ein neues Verfahren zum Herstellen von bekannten fungizid wirksamen 1,3-Dimethyl-5-fluor-4-carboxamiden aus dem entsprechenden Säurefluorid und Anilin-Derivaten in Gegenwart von Alkylpyridin-Derivaten als Säureakzeptor.

Es ist bereits bekannt, dass man 1,3'-Dimethyl-5-fluor-1H-pyrazol-4-carboxamide durch Umsetzung des entsprechenden Säurefluorids mit dem gewünschten Anilin-Derivat erhält (vgl. EP-A 0 776 889). Bevorzugt werden nach dieser Beschreibung bicyclische tertiäre Amine wie Diazabicyclooctan (DABCO), Diazabicyclononen (DBN) oder Diazabicycloundecen (DBU) als Säureakzeptor eingesetzt. Die Umsetzung mit DABCO liefert lediglich eine Ausbeute von 80 %. Außerdem ist DABCO für großtechnische Umsetzungen ungeeignet, das dieses Reagenz sehr teuer und nicht zu recyclieren ist.

Es wurde nun gefunden, dass man Carboxamide der Formel (I) 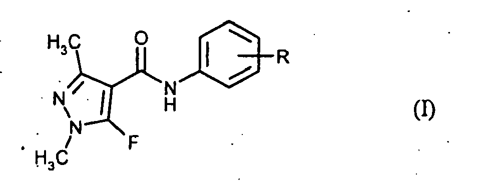 in welcher
- R: für C₃-C₁₂-Cycloalkyl, C₃-C₁₂-Cycloalkenyl, C₆-C₁₂-Bicycloalkyl, C₂-C₁₂-Oxacycloalkyl, C₄-C₁₂-Oxacycloalkenyl, C₃-C₁₂-Thiacycloalkyl, C₄-C₁₂-Thiacycloalkenyl, C₂-C₁₂-Azacy- cloalkyl steht, welche jeweils gegebenenfalls einfach oder mehrfach, gleich oder verschie- den durch C₁-C₈-Alkyl, C₁-C₈-Alkoxy, Halogen und/oder Cyano substituiert sein können, oder für gegebenenfalls einfach bis fünffach, gleich oder verschieden substituiertes Phenyl steht, wobei die Substituenten jeweils aus der Liste W¹ ausgewählt sind, oder für unsubstituiertes C₂-C₂₀-Alkyl, oder für einfach oder mehrfach, gleich oder verschieden durch Halogen, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-al- kyl)amino, C₁-C₆-Halogenalkylthio, C₁-C₆-Halogenalkylsulfinyl, C₁-C₆-Halogenalkylsulfo- nyl, C₁-C₆-Halogenalkoxy, C₁-C₆-Halogenalkylamino, Halogen-di(C₁-C₆-alkyl)amino, -SiR¹R²R³ und/oder C₃-C₆-Cycloalkyl substituiertes C₁-C₂₀-Alkyl steht, wobei der Cyclo- alkylteil seinerseits gegebenenfalls einfach bis vierfach, gleich oder verschieden durch Halogen, C₁-C₄-Alkyl und/oder C₁-C₄-Halogenalkyl substituiert sein kann,
- W¹: für Halogen, Cyano, Nitro, Amino, Hydroxy, C₁-C₈-Alkyl, C₁-C₈-Alkoxy, C₁-C₈-Alkylthio, C₁-C₈-Alkylsulfinyl, C₁-C₈-Alkylsulfonyl, C₂-C₆-Alkenyl, C₂-C₆-Alkenyloxy; C₁-C₆-Halo- genalkyl, C₁-C₆-Halogenalkoxy, C₁-C₆-Halogenalkylthio, C₁-C₆-Halogenalkylsulfinyl oder C₁-C6-Halogenalkylsulfonyl mit jeweils 1 bis 13 gleichen oder verschiedenen Halogen- atomen; C₂-C₆-Halogenalkenyl oder C₂-C₆-Halogenalkenyloxy mit jeweils 1 bis 11 gleichen oder verschiedenen Halogenatomen; C₃-C₆-Cycloalkyl oder C₁-C₆-Cycloalkyloxy;
- R¹ und R²: unabhängig voneinander für Wasserstoff, C₁-C₈-Alkyl, C₁-C₈-Alkoxy, C₁-C₄-Alkoxy-C₁- C₄-alkyl, C₁-C₄-Alkylthio-C₁-C₄-alkyl oder C₁-C₆-Halogenalkyl stehen,
- R³: für Wasserstoff, C₁-C₈-Alkyl, C₁-C₈-Alkoxy, C₁-C₄-Alkoxy-C₁-C₄-alkyl, C₁-C₄-Alkylthio- C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl, C₁-C₆-Cycloalkyl, oder für jeweils gegebenenfalls substituiertes Phenyl oder Phenylalkyl steht,

auf einfache Weise erhält, indem man

5-Fluor-1,3-dimethyl-1H-pyrazol-4-carbonsäurefluorid der Formel (II) 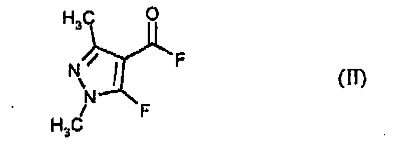 mit Anilin-Derivaten der Formel (III) 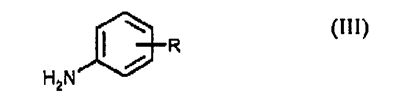 in welcher R die oben angegebenen Bedeutungen hat,
in Gegenwart eines Säureakzeptors der Formel (IV) 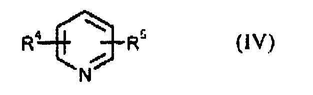 in welcher R⁴ und R⁵ unabhängig voneinander für Methyl oder Ethyl stehen,
um setzt.

Überraschenderweise lassen sich die Carboxamide der Formel (I) unter den erfindungsgemäßen Bedingungen durch die Wahl des Säureakzeptors der Formel (IV) mit guten Ausbeuten in hoher Reinheit und Selektivität herstellen. Ein weiterer Vorteil des erfindungsgemäßen Verfahrens ist, dass sich der eingesetzte Säureakzeptor der Formel (IV) komplett zurückgewonnen werden kann.

Verwendet man beispielsweise 5-Fluor-1,3-dimethyl-1H-pyrazol-4-carbönsäurefluorid und 2-(1,3-Dimethyl-butyl)-phenylamin als Ausgangsstoffe sowie 2,6-Dimethylpyridin als Säureakzeptor, so kann das erfindungsgemäße Verfahren durch das folgende Formelschema veranschaulicht werden:

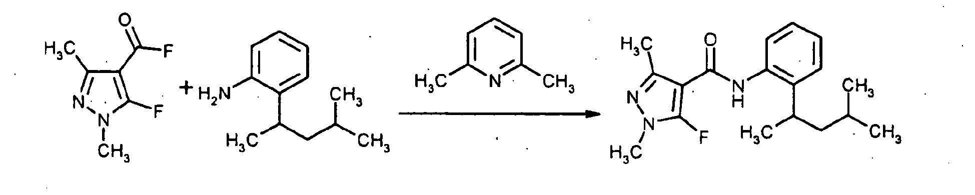

Das bei der Durchführung des erfindungsgemäßen Verfahrens als Ausgangsstoff verwendete 5-Fluor-1,3-dimethyl-1H-pyrazol-4-carbonsäurefluorid der Formel (II) ist bekannt (vgl. EP-A 0 776 889).

Die bei der Durchführung des erfindungsgemäßen Verfahrens weiterhin als Ausgangsstoffe verwendeten Anilin-Derivate sind durch die Formel (III) allgemein definiert.
- R: steht bevorzugt für C₃-C₈-Cycloalkyl, C₃-C₈-Cycloalkenyl, C₆-C₁₀-Bicycloalkyl, C₂-C₇- Oxacycloalkyl, C₄-C₇-Oxacycloalkenyl, C₃-C₇-Thiacycloalkyl, C₄-C₇-Thiacydoalkenyl, C₂- C₇-Azacycloalkyl steht, welche jeweils gegebenenfalls einfach bis vierfach, gleich oder verschieden durch C₁-C₄-Alkyl, C₁-C₄-Alkoxy, Fluor, Chlor, Brom und/oder Cyano substituiert sein können, oder für einfach bis dreifach, gleich oder verschieden substituiertes Phenyl, wobei die Sub- stituenten aus der Liste W¹ ausgewählt sind, oder für unsubstituiertes C₂-C₁₂-Alkyl (wie Ethyl und geradkettiges oder verzweigtes Propyl, Butyl, Pentyl, Hexyl, Heptyl, Octyl, Nonyl, Decyl, Undecyl und Dodecyl) oder für einfach oder mehrfach, gleich oder verschieden durch Fluor, Chlor, Brom, Iod, C₁- C₆-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halo- genalkylsulfonyl, C₁-C₄-Halogenalkoxy, C₁-C₄-Halogenalkylamino, Halogen-di(C₁-C₄-al- kyl)amino mit jeweils 1 bis 9 Fluor-, Chlor- und/oder Bromatomen, -SiR¹R²R³, Cyclopro- pyl, Dichlorcyclopropyl, Cyclobutyl, Cyclopentyl oder Cyclohexyl substituiertes C₁-C₁₂- Alkyl (wie Methyl, Ethyl und geradkettiges oder verzweigtes Propyl, Butyl, Pentyl, Hexyl, Heptyl, Octyl, Nonyl, Decyl, Undecyl und Dodecyl).
- R: steht besonders bevorzugt für Cyclopentyl, Cyclohexyl, Cycloheptyl, Bicyclo[2.2.1]heptyl, oder für einfach in 4-Position substituiertes Phenyl, für zweifach, gleich oder verschieden in 3,4-, 2,3-, 2,4- oder 3,5-Position substituiertes Phenyl oder für dreifach, gleich oder ver- schieden in 2,4,6-Position substituiertes Phenyl, wobei die Substituenten jeweils aus der Liste W¹. ausgewählt sind, oder für unsubstituiertes C₃-C₁₀-Alkyl (wie Propyl, 1-Methylethyl, Butyl, 1-Methylpropyl, 2- Methylpropyl, 1,1-Dimethylethyl, n-Pentyl, 1-Methylbutyl, 2-Methylbutyl, 3-Methylbutyl, 1,2-. Dimethylpropyl, 1,1-Dimethylpropyl, 2,2-Dimethylpropyl, 1-Ethylpropyl, n-Hexyl, 1-Methyl- pentyl, 2-Methylpentyl, 3-Methylpentyl, 4-Methylpentyl, 1,2-Dimethylbutyl, 1,3-Dimethyl- butyl, 2,3-Dimethylbutyl, 1,1-Dimethylbutyl, 2,2-Dimethylbutyl, 3,3-Dimethylbutyl, 1,1,2-Tri- methylpropyl, 1,2,2-Trimethylpropyl, 1-Ethylbutyl, 2-Ethylbutyl, 1-Ethyl-3-methylpropyl, n- Heptyl, 1-Methylhexyl, 1-Ethylpentyl, 2-Ethylpentyl, 1-Propylbutyl, Octyl, 1-Methylheptyl, 2- Methylheptyl, 1-Ethylhexyl, 2-Ethylhexyl, 1-Propylpentyl, 2-Propylpentyl, Nonyl, 1-Methyl- octyl, 2-Methyloctyl, 1-Ethylheptyl, 2-Ethylheptyl, 1-Propylhexyl, 2-Propylhexyl, Decyl, 1- Methylnonyl, 2-Methylnonyl, 1-Ethyloctyl, 2-Ethyloctyl, 1-Propylheptyl und 2-Propylheptyl) oder für einfach oder mehrfach, gleich oder verschieden durch Fluor, Chlor, Methylthio, Ethylthio, n- oder iso-Propylthio, n-, iso-, sec-, tert-Butylthio, Pentylthio, Hexylthio, Me- thylsulfonyl, Ethylsulfonyl, n- oder iso-Propylsulfonyl; n-, iso-, sec-, tert-Butylsulfonyl, Methoxy; Ethoxy, n- oder iso-Propoxy, n-, iso-, sec-, tert-Butoxy, Methylamino, Ethyl- amino, n- oder iso-Propylamino, n-, iso-, sec-, tert-Butylamino, Dimethylamino, Diisopro- pylamino, Trifluormethylthio, Trifluormethoxy, -SiR¹R²R³, Cyclopropyl, Dichlorcyclo- propyl, Cyclobutyl, Cyclopentyl oder Cyclohexyl substituiertes C₁-C₁₀-Alkyl (wie Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dime- thylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbu- tyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-3-methylpropyl, n-Heptyl, 1- Methylhexyl, 1-Ethylpentyl, 2-Ethylpentyl, 1-Propylbutyl, Octyl, 1-Methylheptyl, 2-Me- thylheptyl, 1-Ethylhexyl, 2-Ethylhexyl, 1-Propylpentyl, 2-Propylpentyl, Nonyl, 1-Methyl-- octyl, 2-Methyloctyl, 1-Ethylheptyl, 2-Ethylheptyl, 1-Propylhexyl, 2-Propylhexyl, Decyl, 1-Methylnonyl, 2-Methylnonyl, 1-Ethyloctyl, 2-Ethyloctyl, 1-Propylheptyl und 2-Propylheptyl).
- W¹: steht bevorzugt für Fluor, Chlor, Brom, Methyl, Ethyl, n- oder i-Propyl, n-, i-, s- oder t- Butyl, Methoxy, Ethoxy, n- oder i-Propoxy, Trifluormethyl, Trifluorethyl, Difluor- methoxy, Trifluormethoxy, Diflüorchlormethoxy, Trifluorethoxy.
- W¹: steht besonders bevorzugt Fluor, Chlor oder Brom.

- R' und R²: stehen unabhängig voneinander bevorzugt für C₁-C₆-Alkyl, C₁-C₆-Alkoxy, C₁-C₃- Alkoxy-C₁-C₃-alkyl oder C₁-C₃-Alkylthio-C₁-C₃-alkyl.
- R¹ und R²: stehen unabhängig voneinander besonders bevorzugt für Methyl, Ethyl, Methoxy, Ethoxy, Methoxymethyl, Ethoxymethyl, Methoxyethyl, Ethoxyethyl, Methylthiomethyl, Ethylthiomethyl, Methylthioethyl oder Ethylthioethyl.
- R¹ und R²: stehen unabhängig voneinander ganz besonders bevorzugt für Methyl, Methoxy, Methoxymethyl oder Methylthiomethyl.
- R¹ und R²: stehen insbesondere bevorzugt jeweils für Methyl.
- R³: steht Bevorzugt für C₁-C₆-Alkyl, C₁-C₆-Alkoxy, C₁-C₃-Alkoxy-C₁-C₃-alkyl, C₁-C₃- Alkylthio-C₁-C₃-alkyl, C₃-C₆-Cycloalkyl, Phenyl oder Benzyl.
- R³: steht besonders bevorzugt für Methyl, Ethyl, n- oder iso-Propyl, n-, sec-, iso- oder tert- Butyl, Methoxy, Ethoxy, n- oder iso-Propoxy, n-, sec-, iso- oder tert-Butoxy, Methoxymethyl, Ethoxymethyl, Methoxyethyl, Ethoxyethyl, Methylthiomethyl, Ethylthio- methyl, Methylthioethyl, Ethylthioethyl, Cyclopropyl, Phenyl oder Benzyl.
- R³: steht ganz besonders bevorzugt für Methyl, Ethyl, n- oder iso-Propyl, iso- oder tert-Butyl, Methoxy; iso-Propoxy, iso- oder tert-Butoxy.
- R³: steht insbesondere bevorzugt für Methyl.

Bevorzugt werden Anilin-Derivate der Formel (III-1) 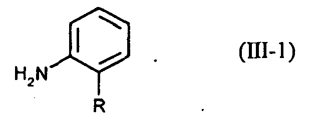 in welcher R die oben angegebenen Bedeutungen hat,
in das erfindungsgemäße Verfahren eingesetzt.

Außerdem bevorzugt werden Anilin-Derivate der Formel (III-2) 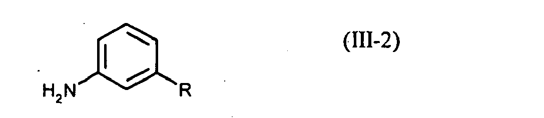 in welcher R die oben angegebenen Bedeutungen hat,
in das erfindungsgemäße Verfahren eingesetzt.

Außerdem bevorzugt werden Anilin-Derivate der Formel (III-3) 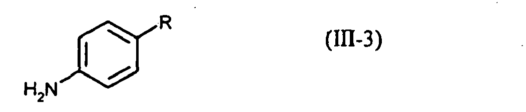 in welcher R die oben angegebenen Bedeutungen hat,
in das erfindungsgemäße Verfahren eingesetzt.

Besonders bevorzugt setzt man Anilin-Derivate der Formel (ill-1) ein.

Anilin-Derivate der Formel (III) bzw. (III-1), (III-2) und (III-3) sind bekannt oder lassen sich auf bekannte Weise herstellen (vgl. EP-A 0 776 889, WO 03/010149).

Die bei der Durchführung des erfindungsgemäßen Verfahrens verwendeten Säureakzeptoren sind durch die Formel (IV) allgemein definiert. In dieser Formel (IV) stehen R⁴ und R⁵ unabhängig voneinander bevorzugt für Methyl oder Ethyl. Besonders bevorzugt sind folgende Säureakzeptoren der Formel (IV): 2-Methylpyridin, 2,3-Dimethlypyridin, 2-Methyl-5-ethylpyridin, 2,6-Dimethylpyridin, 2,4-Dimethylpyridin, 3,4-Dimethylpyridin, 2,4,6-Trimethlypyridin.

Säureakzeptoren der Formel (IV) sind bekannte Synthesechemikalien.

Das erfindungsgemäße Verfahrens kann in Gegenwart eines Verdünnungsmittels durchgeführt werden. Hierzu kommen alle inerten organischen Lösungsmittel in Betracht, vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid, besonders bevorzugt Chlorbenzol oder Toluol.

Die Reaktionstemperaturen können bei der Durchführung des erfindungsgemäßen Verfahrens in einem größeren Bereich variiert werden. Im Allgemeinen arbeitet man bei Temperaturen von 100°C bis 150°C, bevorzugt bei Temperaturen von 120°C bis 145°C.

Bei der Durchführung des erfindungsgemäßen Verfahrens setzt man pro Mol des 5-Fluor-1,3-dimethyl-1H-pyrazol-4-carbonsäurefluorids der Formel (II) im Allgemeinen zwischen 0,8 und 1,5 Mol, bevorzugt äquimolare Mengen, von Anilin-Derivaten der Formel (III) sowie zwischen 0,8 und 1,5 Mol, bevorzugt äquimolare Mengen, eines Säureakzeptors der Formel (IV) ein.

Die Reaktionszeit kann in Abhängigkeit von der Reaktivität der Edukte bis zu 30 Stunden betragen, wobei der Abbruch der Reaktion bei vollständigem Umsatz auch schon früher erfolgen kann. Bevorzugt sind Reaktionszeiten von 10 bis 20 Stunden.

Alle erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die durch das erfindungsgemäßen Verfahren herstellbaren Carboxamide der Formel (I) sind wertvolle Fungizide (vgl. z.B. WO 03/010149).

Das erfindungsgemäße Herstellen von Carboxamiden der Formel (I) wird in den nachstehenden Beispielen beschrieben, welche die obige Beschreibung weiter illustrieren. Die Beispiele sind jedoch nicht in einschränkender Weise zu interpretieren.

### Herstellungsbeispiele

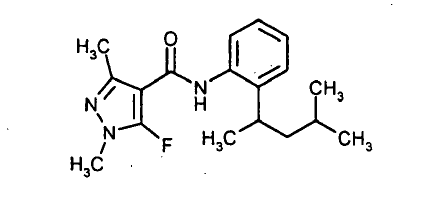

Unter Schutzgas (Argon) wird eine Lösung aus 11,25 g (105 mmol) 2,6-Dimethylpyridin und 18,05 g (100 mmol) 2-(1,3-Dimethyl-butyl)-phenylamin in 40 ml Chlorbenzol vorgelegt. Man gibt 16,17 g (100 mmol) 5-Fluor-1,3-dimethyl-1H-pyrazol-4-carbonsäurefluorid zu und rührt für 21 h unter Rückfluss nach. Zur Aufarbeitung lässt man abkühlen, verrührt mit 100 ml 1 N Salzsäure und extrahiert dreimal mit je 100 ml Essigsäureethylester. Die vereinigten organischen Phasen werden einmal mit 100 ml Wasser gewaschen, über Magnesiumsulfat getrocknet und unter vermindertem Druck eingeengt. Die erhaltene Suspension wird mit 50 ml n-Hexan für 2 h bei Raumtemperatur nachgerührt. Man erhält 28,25 g (88 % der Theorie) an N-[2-(1,3-Dimethylbutyl)phenyl]-5-fluor-1,3-dimethyl-1H-pyräzol-4-carboxamid in Form von Kristallen (Schmelzpunkt 104-106°C).

## Patentansprüche

1. Verfahren zum Herstellen von Carboxamiden der Formel (I) 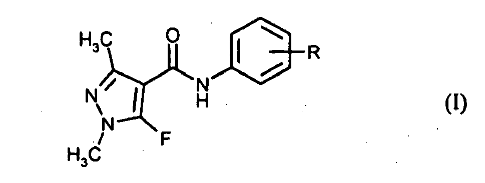 in welcher
R für C₃-C₁₂-Cycloalkyl, C₃-C₁₂-Cycloalkenyl, C₆-C₁₂-Bicycloalkyl, C₂-C₁₂-Oxacy- cloalkyl, C₄-C₁₂-Oxacycloalkenyl, C₃-C₁₂-Thiacycloalkyl, C₄-C₁₂-Thiacycloalkenyl, C₂-C₁₂-Azacycloalkyl steht, welche jeweils gegebenenfalls einfach oder mehrfach, gleich oder verschieden durch C₁-C₈-Alkyl, C₁-C₈-Alkoxy, Halogen und/oder Cyano substituiert sein können, oder für gegebenenfalls einfach bis fünffach, gleich oder verschieden substituiertes Phenyl steht, wobei die Substituenten jeweils aus der Liste W¹ ausgewählt sind, oder für unsubstituiertes C₂-C₂₀-Alkyl, oder für einfach oder mehrfach, gleich oder verschieden durch Halogen, C₁-C₆-Al- kylthio, C₁-C₆-Alkylsulfinyl, C₁-C₆-Alkylsulfonyl, C₁-C₆-Alkoxy, C₁-C₆-Alkylami- no, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkylamino, -SiR¹R²R³ und/oder C₃-C₆-Cycloalkyl substituier- tes C₁-C₂₀-Alkyl steht, wobei der Cycloalkylteil seinerseits gegebenenfalls einfach bis vierfach, gleich oder verschieden durch Halogen, C₁-C₄-Alkyl und/oder C₁-C₄- Halogenalkyl substituiert sein kann,
W¹ für Halogen, Cyano, Nitro, Amino, Hydroxy, C₁-C₈-Alkyl, C₁-C₈-Alkoxy, C₁-C₈- Alkylthio, C₁-C₈-Alky)sulfinyl, C₁-C₈-Alkylsulfonyl, C₂-C₆-Alkenyl, C₂-C₆- Alkenyloxy; C₁-C₆-Halogenalkyl, C₁-C₆-Halogenalkoxy, C₁-C₆-Halogenalkylthio, C₁-C₆-Halogenalkylsulfinyl oder C₁-C₆-Halogenalkylsulfonyl mit jeweils 1 bis 13 gleichen oder verschiedenen Halogenatomen; C₂-C₆-Halogenalkenyl oder C₂-C₆- Halogenalkenyloxy mit jeweils 1 bis 11 gleichen oder verschiedenen Halogen- atomen; C₃-C₆-Cycloalkyl oder C₃-C₆-Cycloalkyloxy;
R¹ und R² unabhängig voneinander für Wasserstoff, C₁-C₈-Alkyl, C₁-C₈-Alkoxy, C₁-C₄- Alkoxy-C₁-C₄-alkyl, C₁-C₄-Alkylthio-C₁-C₄-alkyl oder C₁-C₆-Halogenalkyl stehen,
R³ für Wasserstoff, C₁-C₈-Alkyl, C₁-C₈-Alkoxy, C₁-C₄-Alkoxy-C₁-C₄-alkyl, C₁-C₄- Alkylthio-C₁-C₄-alkyl, C₂-C₈-Alkenyl, C₂-C₈-Alkinyl, C₁-C₆-Halogenalkyl, C₂-C₆- Halogenalkenyl, C₂-C₆-Halogenalkinyl, C₃-C₆-Cycloalkyl, oder für jeweils gegebe- nenfalls substituiertes Phenyl oder Phenylalkyl steht,
**dadurch gekennzeichnet, dass** man
5-Fluor-1,3-dimethyl-1H-pyrazol-4-carbonsäurefluorid der Formel (II) 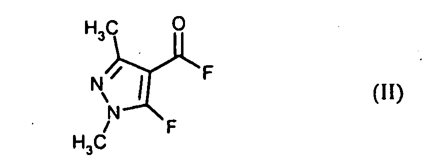 mit Anilin-Derivaten der Formel (III) 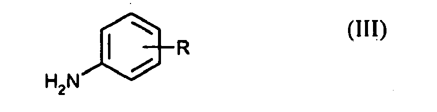 in welcher R die oben angegebenen Bedeutungen hat,
in Gegenwart eines Säureakzeptors der Formel (IV) 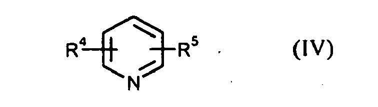 in welcher R⁴ und R⁵ unabhängig voneinander für Methyl oder Ethyl stehen,
umsetzt.

2. Verfahren gemäß Anspruch 1, **dadurch gekennzeichnet, dass** man als Säureakzeptor 2-Methylpyridin, 2,3-Dimethlypyridin, 2-Methyl-5-ethylpyridin, 2,6-Dimethylpyridin, 2,4-Dimethylpyridin, 3,4-Dimethylpyridin, 2,4,6-Trimethlypyridin einsetzt.

3. Verfahren gemäß Anspruch 1, **dadurch gekennzeichnet, dass** man als Säureakzeptor 2,6-Dimethylpyridin einsetzt.

4. Verfahren gemäß Anspruch 1, 2 oder 3, **dadurch gekennzeichnet, dass** man bei Temperaturen von 100°C bis 150°C arbeitet.

5. Verfahren gemäß Anspruch 1, 2, 3 oder 4, **dadurch gekennzeichnet, dass** man Anilin-Derivate der Formel (III-1) 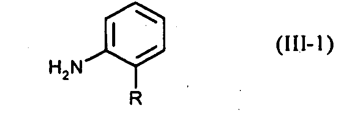 in welcher R die in Anspruch 1 angegebenen Bedeutungen hat, einsetzt.

6. Verfahren gemäß Anspruch 1, 2, 3, 4 oder 5, **dadurch gekennzeichnet, dass** man 2-(1,3-Dimethyl-butyl)-phenylamin als Anilin-Derivat der Formel (III) einsetzt.

7. Verfahren gemäß Anspruch 1, 2, 3, 4, 5 oder 6, **dadurch gekennzeichnet, dass** man 5-Fluor-1,3-dimethyl-1H-pyrazol-4-carbonsäurefluorid der Formel (II) 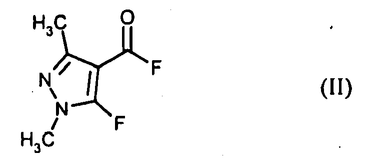 mit 2-(1,3-Dimethyl-butyl)-phenylamin als Anilin-Derivat der Formel (III) in Gegenwart von 2,6-Dimethylpyridin als Säureakzeptor umsetzt.

## Claims

1. Process for preparing carboxamides of the formula (I) 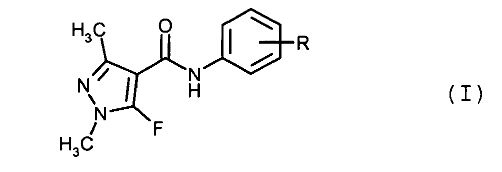 in which
R is C₃-C₁₂-cycloalkyl, C₃-C₁₂-cycloalkenyl, C₆- C₁₂-bicycloalkyl, C₂-C₁₂-oxacycloalkyl, C₄-C₁₂- oxacycloalkenyl, C₃-C₁₂-thiacycloalkyl, C₄-C₁₂- thiacycloalkenyl, C₂-C₁₂-azacycloalkyl, each of which may optionally be mono- or polysubstituted identically or differently by C₁-C₈-alkyl, C₁-C₈-alkoxy, halogen and/or cyano, or phenyl which is optionally mono- to pentasubstituted identically or differently, where the substituents are each selected from the list W¹, or unsubstituted C₂-C₂₀-alkyl, or C₁-C₂₀-alkyl which is mono- or polysubstituted identically or differently by halogen, C₁-C₆-alkylthio, C₁-C₆-alkylsulfinyl, C₁-C₆-alkylsulfonyl, C₁-C₆-alkoxy, C₁-C₆-alkyl- amino, di (C₁-C₆-alkyl) amino, C₁-C₆-haloalkyl- thio, C₁-C₆-haloalkylsulfinyl, C₁-C₆-haloalkyl- sulfonyl, C₁-C₆-haloalkoxy, C₁-C₆-halo- alkylamino, halo-di(C₁-C₆-alkyl)amino, -SiR¹R²R³ and/or C₃-C₆-cycloalkyl, where the cycloalkyl moiety may in turn optionally be mono- to tetrasubstituted identically or differently by halogen, C₁-C₄-alkyl and/or C₁- C₄-haloalkyl,
W¹ is halogen, cyano, nitro, amino, hydroxyl, C₁- C₈-alkyl, C₁-C₈-alkoxy, C₁-C₈-alkylthio, C₁-C₈- alkylsulfinyl, C₁-C₈-alkylsulfonyl, C₂-C₆- alkenyl, C₂-C₆-alkenyloxy; C₁-C₆-haloalkyl, C₁- C₆-haloalkoxy, C₁-C₆-haloalkylthio, C₁-C₆-halo- alkylsulfinyl or C₁-C₆-haloalkylsulfonyl having in each case from 1 to 13 identical or different halogen atoms; C₂-C₆-haloalkenyl or C₂-C₆-haloalkenyloxy having in each case from 1 to 11 identical or different halogen atoms; C₃-C₆-cycloalkyl or C₃-C₆-cycloalkyloxy;
R¹ and R² are each independently hydrogen, C₁-C₈- alkyl, C₁-C₈-alkoxy, C₁-C₄-alkoxy-C₁-C₄-alkyl, C₁-C₄-alkylthio-C₁-C₄-alkyl or C₁-C₆-haloalkyl,
R³ is hydrogen, C₁-C₈-alkyl, C₁-C₈-alkoxy, C₁-C₄- alkoxy-C₁-C₄-alkyl, C₁-C₄-alkylthio-C₁-C₄- alkyl, C₂-C₈-alkenyl, C₂-C₈-alkynyl, C₁-C₆- haloalkyl, C₂-C₆-haloalkenyl, C₂-C₆-halo- alkynyl, C₃-C₆-cycloalkyl, or in each case optionally substituted phenyl or phenylalkyl,
**characterized in that**
5-fluoro-1,3-dimethyl-1H-pyrazole-4-carbonyl fluoride of the formula (II) 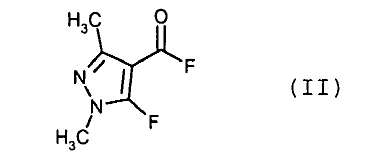 is reacted with aniline derivatives of the formula (III) 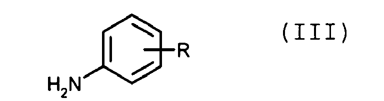 in which R is as defined above,
in the presence of an acid acceptor of the formula (IV) 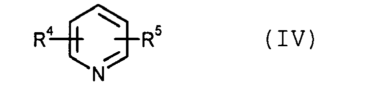 in which R⁴ and R⁵ are each independently methyl or ethyl.

2. Process according to Claim 1, **characterized in that** the acid acceptor used is 2-methylpyridine, 2,3-dimethylpyridine, 2-methyl-5-ethylpyridine, 2,6-dimethylpyridine, 2,4-dimethylpyridine, 3,4-dimethylpyridine, 2,4,6-trimethylpyridine.

3. Process according to Claim 1, **characterized in that** the acid acceptor used is 2,6-dimethylpyridine.

4. Process according to Claim 1, 2 or 3, **characterized in that** temperatures of from 100 to 150°C are employed.

5. Process according to Claim 1, 2, 3 or 4, **characterized in that** aniline derivatives of the formula (III-1) 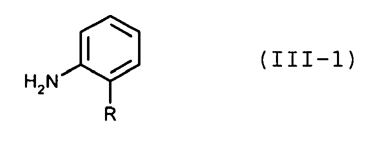 in which R is as defined in Claim 1 are used.

6. Process according to Claim 1, 2, 3, 4 or 5, **characterized in that** 2-(1,3-dimethylbutyl)-phenylamine is used as the aniline derivative of the formula (III).

7. Process according to Claim 1, 2, 3, 4, 5 or 6, **characterized in that** 5-fluoro-1,3-dimethyl-1H-pyrazole-4-carbonyl fluoride of the formula (II) 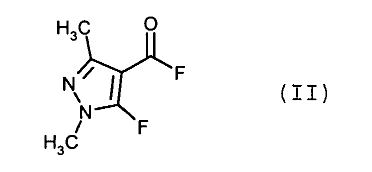 is reacted with 2-(1,3-dimethylbutyl)phenylamine as the aniline derivative of the formula (III) in the presence of 2,6-dimethylpyridine as the acid acceptor.

## Revendications

1. Procédé de production de carboxamides de la formule (I) 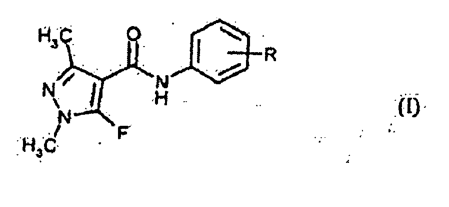 dans laquelle
R représente un cycloalkyle(C₃-C₁₂), un cyclo- alcényle(C₃-C₁₂), un bicycloalkyle(C₆-C₁₂), un oxacycloalkyle(C₂-C₁₂), un oxacycloalcényle(C₄-C₁₂), un thiacycloalkyle(C₃-C₁₂), un thiacycloalcényle(C₄- C₁₂), un azacycloalkyle(C₂-C₁₂), qui peuvent être respectivement substitués éventuellement de façon simple ou multiple, identique ou différente, par un alkyle(C₁-C₈), un alcoxy(C₁-C₈), un halogène et/ou un cyano, ou représente un phényle, substitué éventuellement de façon simple à quintuple, identique ou différente, dans lequel les substituants sont respectivement choisis dans la liste W¹, ou représente un alkyle(C₂-C₂₀) non substitué, ou représente un alkyle(C₁-C₂₀) substitué de façon simple ou multiple, identique ou différente, par un halogène, un alkylthio(C₁-C₆), un alkylsulfinyle(C₁- C₆), un alkylsulfonyle(C₁-C₆), un alcoxy(C₁-C₆), un
W¹ alkylamino(C₁-C₆), un di (alkyle(C₁-C₆)) amino, un halogénoalkylthio(C₁-C₆), un halogénoalkyl- sulfinyle(C₁-C₆), un halogénoalkylsulfonyle(C₁-C₆), un halogénoalcoxy(C₁-C₆), un halogénoalkylamino(C₁-C₆), un halogénodi(alkyl (C₁-C₆))amino, un -SiR¹R²R³ et/ou un cycloalkyle(C₃-C₆), dans lequel la fraction cycloalkyle peut de son côté être substituée, éventuellement de façon simple à quadruple, identique ou différente, par un halogène, un alkyle (C₁-C₉) et/ou un halogénoalkyle(C₁-C₉), représente un halogène, un cyano, un nitro, un amino, un hydroxy, un alkyle(C₁-C₈), un alcoxy(C₁-C₈), un alkylthio(C₁-C₈), un alkylsylfinyle(C₁-C₈), un alkylsulfonyle(C₁-C₈), un alcényle(C₂-C₆), un alcényloxy(C₂-C₆), un halogénoalkyle(C₁-C₆) un halogénoalcoxy(C₁-C₆), un halogénoalkylthio(C₁-C₆), un halogénoalkylsulfinyle(C₁-C₆) ou un halogéno- alkylsulfonyle(C₁-C₆), avec respectivement 1 à 13 atomes de halogène identiques ou différents, un halogénoalcényle(C₂-C₆) ou un halogénoalcényloxy(C₂- C₆) avec respectivement 1 à 11 atomes de halogène identiques ou différents, un cycloalkyle(C₃-C₆) ou un cycloalkyloxy(C₃-C₆);
R¹ et R² représentent, indépendamment l'un de l'autre, un hydrogène, un alkyle(C₁-C₈), un alcoxy(C₁-C₈), un alcoxy(C₁-C₄)-alkyle(C₁-C₄), un alkylthio(C₁-C₄)- alkyle(C₁-C₄) ou un halogénoalkyle(C₁-C₆) ;
R³ représente un hydrogène, un alkyle(C₁-C₈), un alcoxy(C₁-C₈), un alcoxy(C₁-C₄)-alkyle(C₁-C₄), un alkylthio(C₁-C₄)-alkyle(C₁-C₄), un alcényle(C₂-C₈), un alcinyle(C₂-C₈), un halogénoalkyle(C₁-C₆), un halogénoalcényle(C₂-C₆), un halogénoalcinyle(C₂-C₆), un cycloalkyle(C₃-C₆), ou un phényle ou phénylalkyle chaque fois éventuellement substitué,
**caractérisé en ce que** l'on convertit
un fluorure de l'acide 5-fluor-1,3-diméthyl-1H-pyrazol-4-carboxylique de la formule (II) 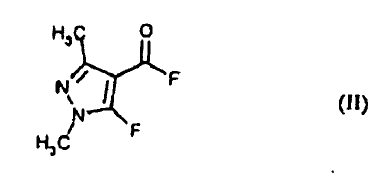 avec des dérivés de l'aniline de la formule (III) 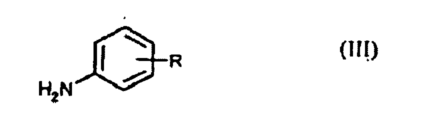 dans laquelle R a les significations indiquées plus haut,
en présence d'un accepteur d'acide de la formule (IV) 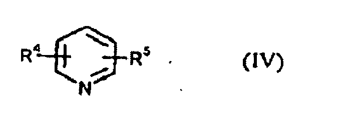 dans laquelle R⁴ et R⁵ représentent indépendamment l'un de l'autre un méthyle ou un éthyle.

2. Procédé selon la revendication 1, **caractérisé en ce que** l'on utilise comme accepteur d'acide la 2-méthylpyridine, la 2,3-diméthylpyridine, la 2-méthyl-5-éthylpyridine, la 2,6-diméthylpyridine, la 2,4-diméthylpyridine, la 3,4-diméthylpyridine, la 2,4,6-triméthylpyridine.

3. Procédé selon la revendication 1, **caractérisé en ce que** l'on utilise comme accepteur d'acide la 2,6-diméthylpyridine.

4. Procédé selon la revendication 1, 2 ou 3, **caractérisé en ce que** l'on travaille à des températures de 100°C à 150°C.

5. Procédé selon la revendication 1, 2, 3 ou 4, **caractérisé en ce que** l'on utilise des dérivés de l'aniline de la formule (III-1) 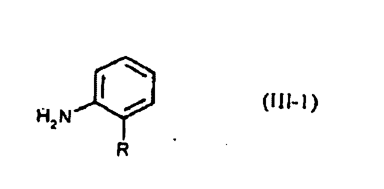 dans laquelle R a les significations indiquées dans la revendication 1.

6. Procédé selon la revendication 1, 2, 3, 4 ou 5, **caractérisé en ce que** l'on utilise la 2-(1,3-diméthylbutyl)-phénylamine comme dérivé de l'aniline de la formule (III).

7. Procédé selon la revendication 1, 2, 3, 4, 5 ou 6, **caractérisé en ce que** l'on convertit le fluorure de l'acide 5-fluor-1,3-diméthyl-1H-pyrazol-4-carboxylique de la formule (II) 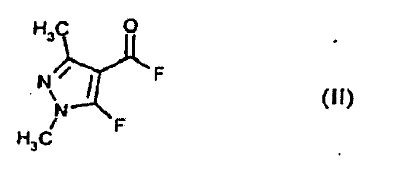 avec la 2-(1,3-diméthyl-butyl)-phénylamine comme dérivé de l'aniline de la formule (III) en présence de 2,6-diméthylpyridine comme accepteur d'acide.
